# EUROPEAN PATENT APPLICATION

(11) **EP 1 972 261 A1**
(43) Date of publication of application: **24.09.2008**
(21) Application number: 08152973.7
(22) Date of filing: 19.03.2008
(51) Int. Cl.: A61B 1/04, A61B 1/12, A61B 1/24, A61B 1/247, A61B 1/253

(54) **Improved device for dental operations**

(30) Priority: 20.03.2007 IT MI20070544
(71) Applicant: F.A.R.O. FABBRICA APPARECCHIATURE RAZIONALI ODONTOIATRICHE S.p.A., 20060 Ornago (Milano) (IT)
(72) Inventor: Favonio, Angelo, 20059 Vimercate (Milan) (IT)
(74) Representative: Martegani, Franco

(57) **Abstract**

A device for medical use and particularly for dental use, of which the main component is a rigid video-endoscope (10) supported by an articulated and compensated arm, comprises:
- a rigid video-endoscope (10) with CCD catcher (13), composed of a grip (11) which is extended into an optical tube (12) coaxial therewith, said optical tube (12) being made so to provide an image of the object examined on the plane of the CCD catcher (13), the imaging being directed in the extension of the axis (14) of the video-endoscope (10);
- a sleeve (32) which covers the optical tube (12) and which protects it from the risks of contamination during use, the sleeve (32) being coaxial with optical tube (12), freely rotatable on itself and lockable in the distal part of the grip (11) of the video-endoscope (10), between the sleeve (32) and the optical tube (12) an airspace being defined in which pressurised air is inserted;
- a compensated, articulated arm that bears, fixed to its distal end, a support on which the video-endoscope (10) is locked. According to the invention, the rotation and reflection of the image is obtained by means of an optical group composed of a first optical half-prism (16), a second optical half-prism (17) and a reflecting body (18) arranged on a rotating housing (19), actuated by a micromotor (22).

## Description

The present invention refers to dentistry and in particular to the dental operation equipment in which the main component consists of a rigid, direct vision video-endoscope installed on an articulated and compensated arm. Such device is destined for mainly but not exclusively dental operations, and permits the dental surgeon to work in control on video screen and carries out the functions of an intra-oral and extra-oral video camera, of a directable lighted mirror, which advantageously replaces in some cases the polymerising lamp and the scialytic lamp.

Through appropriate optional accessories, the device can also carry out the functions of transilluminator, negatoscope, as well as saliva suction pump.

With the oral cavity video cameras of the prior art, the greatest difficulties for the dental surgeon who works with the video-screen derive, on the one hand, from the difficulties of synchronising one's own movements with the image on the screen, and on the other hand from the difficulty in focusing the image itself on the tooth upon which he operates.

It must be kept in mind that in order to work in comfort on only one tooth while only looking at its image transmitted on the control video screen, it is necessary on the one hand that the image of the tooth on the screen is directed in the same way as what the dentist usually sees, with or without his small hand-held mirror, in the patient's mouth, and on the other hand that the image of the tooth sits in a fixed position on the screen, and only the image of the tool moved by the dentist must have the possibility of moving around the image of the tooth in order to end up with the image of the point upon which the dentist must operate.

In order to reach such point, the dentist must therefore move the image-taking aperture of the video-endoscope so that the image of the field of vision on the control video-screen simultaneously comprises the part of the tooth to be treated and the image of the distal end of the work tool which he holds, without the image of the tool blocking that of the point to be reached on the tooth to be treated.

Moreover, the current state of the art does not allow images of the distal faces of teeth - i.e. those faces furthest from the image-taking point - to be taken with a direct vision video-endoscope, at the base of the patient's oral cavity. This is not currently possible due to the size of the rigid video-endoscopes available on the market. Therefore, it is necessary to resort to using deviated view, rigid video-endoscopes, according to the known art.

When the dental surgeon manoeuvres a deviated vision video-endoscope to move the image-taking aperture, in order to find the best imaging angle to work on a tooth, the angle made by the plane of the CCD (the abbreviation commonly used to indicate the generic electronic device for the acquisition of dynamic images, indicated below for the sake of simplicity) of the video-endoscope with the plane of the field of vision continually changes, with the result that, on the control screen the directing of the images of the observed tooth is modified and the dentist can lose his references, thus finding it very difficulty to position the image of the end of his tool on the image of the precise point of the tooth which he tries to reach.

Such a change in directing the image of the tooth on the control screen, between two positions of the image-taking aperture, is the result of the product of a rotation and a homothety, i.e. of a biunique correspondence between segments of superimposing planes. Such homothety is direct or indirect depending on the respective relative positions of the distal end of the tool which the dental surgeon is holding, of the tooth having its image taken and of the image-taking aperture of the video-endoscope.

It follows from this that, when the dentist who operates on a tooth using video control moves the image-taking aperture - from a given working angle - in order to find a different angle of view, if he wishes to continue his work in favourable conditions he must also re-establish the position of the tooth in the direction that it previously occupied. For this, it is necessary that the new image of the tooth that appears on the screen is made to undergo an opposite displacement, i.e. the inverse product of the rotation and the homothety that modified the position of the tooth image, passing from the starting position to the end position of the image-taking aperture.

According to European patent No. 1355570, on behalf of the same applicant, a device is proposed aimed to permit the user to carry out such rotation and such homothety in favourable conditions, due to the combined action of a micromotor which makes the CCD rotate around its axis and an electronic plate which inverts the direction of the signals at the CCD level, that consequently causes the inverting of the images from left to right and from bottom to top. The European patent 1355570, moreover, proposes a device that permits the dentist to easily operate using video control, preventing the image-taking aperture from misting up or the aperture from being blocked by splashes of water or solid particles that are deposited therein. In fact, the device proposed according to said patent provides the distal element of the endoscope, on the image-taking aperture, with a suitable air flow even in the presence of the rotation thereon of the sleeve-tube.

Moreover, in order to ensure that the image of the object is displayed in fixed position on the screen, the device according to the European patent No. 1355570 proposes associating the video-endoscope with an articulated and compensated arm on which said video-endoscope is locked with a pawl.

Finally, in order to affront the ergonomic and economical problems faced by the dental surgeon by the many apparatuses around the chair, the device proposed according to the European patent No. 1355570 can be made so as to permit on the one hand working by using video control and on the other hand examining teeth with the transillumination method, and in addition permit the dentist to use it as:
- a scialytic lamp for more easily lighting the distal zones of the patient's teeth, reducing the operating times;
- as a small mirror without misting up;
- as a negatoscope, that is for reading the radiographs on the control screen;
- as a lamp for polymerising;
- as a surgical suction, thus improving the ergonomics of the dental office, reducing the number of apparatuses around the patient's chair, improving the dentist's work conditions and reducing the investments, and therefore ensuring the patient a better quality of treatment.

In particular, the device according to the European patent No. 1355570 comprises:
A- a rigid video-endoscope with CCD image-taking device, with load storage and direct vision, composed of an grip elongated with an optical tube, the video-endoscope being connected to a control screen;
B- a sleeve tube rotating indefinitely on itself, slidable on the optical tube and lockable with a pawl to the distal part with respect to the grip of the video-endoscope;
C- A compensated, articulated arm.
D- An electronic plate that manages the rotation/inversion of the top/bottom and left/right images.

In practice, the device according to the European patent No. 1355570, while meeting the needs for which it was proposed, has a structure which has proven to be difficult to make and maintain.

The object of the present invention is therefore that of making a device that permits overcoming the limits of the devices according to the known technology and obtaining the previously described technical results.

A further object of the invention is that said device can be made with substantially limited costs, both regarding the production costs and concerning the maintenance costs.

Not least object of the invention is that of making a device that is substantially simple, safe and reliable.

These objectives are achieved according to the present invention by proposing a device defined, in its essential components, in the first claim. The preferred variants and embodiments of the device are specified and defined in the dependent claims.

In particular, on the video-endoscope according to the present invention, the deviated vision is obtained with a sleeve tube that completely encircles the optical tube and can rotate with respect thereto.

Such sleeve can be provided with a small mirror that permits angled vision.

The device according to the present invention permits the user to carry out the reverse transformation necessary for resolving the problems due to the dystonia between the screen display of the image and the movements carried out by the dentist, operating a reverse transformation which is obtained in a very simple manner, that is by rotating the image with respect to the CCD by means of an optical prism and reflecting the image by means of electronic image processing.

Moreover, according to the present invention, in order to easily operate using video control, avoiding that the optical elements are misted or dirtied by water splashes or solid particles, the device according to the present invention provides for a more effective air flow system in order to keep the field of vision clean.

Finally, in order to avoid contamination problems, the optical tube is always protected by sleeves during use. Such sleeves are fixed to the endoscope by means of a quick coupling, they can be autoclaved and are easily replaceable.

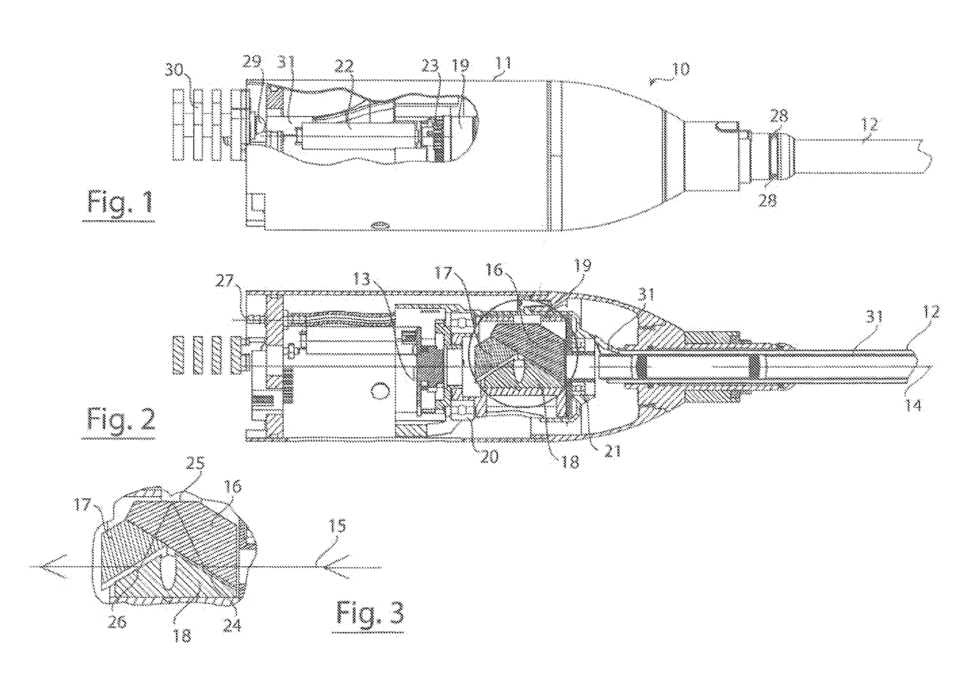

In order to illustrate the particular nature and advantages of the invention, a typical embodiment thereof is illustrated here, as a non-limiting example. In particular, figure 1 illustrates a side view of the direct vision rigid video-endoscope according to the present invention.

Figure 2 illustrates a longitudinal section of the rigid video-endoscope of figure 1.

Figure 3 illustrates a detail of the motorised optical prism for the rotation of the image with respect to the CCD.

Figure 4 illustrates a side view of the distal portion of the rigid video-endoscope of figure 1, inserted in a protection sleeve.

Figure 5 illustrates a longitudinal section of the video-endoscope and sleeve of figure 4.

Figure 6 illustrates the detail A of figure 5.

Figure 7 illustrates the detail B of figure 5.

Figure 8 illustrates the detail C of figure 5.

Figure 9 illustrates a side view of a first embodiment of the protection sleeve of the device according to the present invention.

Figure 10 illustrates a side view of protection sleeve of figure 9, rotated 90°.

Figure 11 illustrates a side view of a second embodiment of the protection sleeve of the device according to the present invention.

Figure 12 illustrates a side view of the protection sleeve of figure 11, rotated 90°.

First making reference to figures 1-3, the device according to the present invention comprises a video-endoscope, indicated overall with the numeric reference 10, comprising a main body or grip 11 that is extended into an optical tube 12 coaxial therewith. The grip 11 also contains a CCD 13 catcher coaxial with the optical tube 12.

The optical tube 12 is made with limited diameter in order to permit being easily inserted in the oral cavity. The optical tube 12 is made to provide, an image of an examined object on the plane of the CCD catcher 13, for example a tooth, not shown. The image-taking is directed in the extension of the axis 14 of the video-endoscope 10. The video screen to which the video-endoscope 10 is connected is not shown in the drawings. The video-endoscope 10 has the characteristic that along the optical axis 15, coinciding with the axis 14 of the video-endoscope 10, between the optical tube 12 and the plane of the CCD catcher 13, an optical group is present composed of a first optical half-prism 16, a second optical half-prism 17 and a reflecting body 18 arranged on a rotating housing 19.

The image of the examined object is reflected from the reflecting surface 24, then from the reflecting surface 25 of the first optical half-prism 16 and from the reflecting surface 26 of the reflecting body 18.

In particular, the rotating housing 19 is housed on a rear bearing 20 and a front bearing 21 and is brought into rotation by a micromotor 22, equipped with a gear head 23.

The rotation of the optical group around its axis permits the inversion of the images.

An air flow runs inside the grip 11, such air flow coming from a generator, not shown, upstream of the device. The air flow enters in the grip 11 through the entrance tube 27 and exits from the grip 11 from small holes 28 made in its distal end, at the base of the optical tube 12.

Inside the body of the video-endoscope, an LED 29 is also arranged, equipped with a radiator 30, in front of which an optical fibre tube 31 is positioned. Such optical fibres 31 convey the light of the LED to the distal end of the optical tube 12.

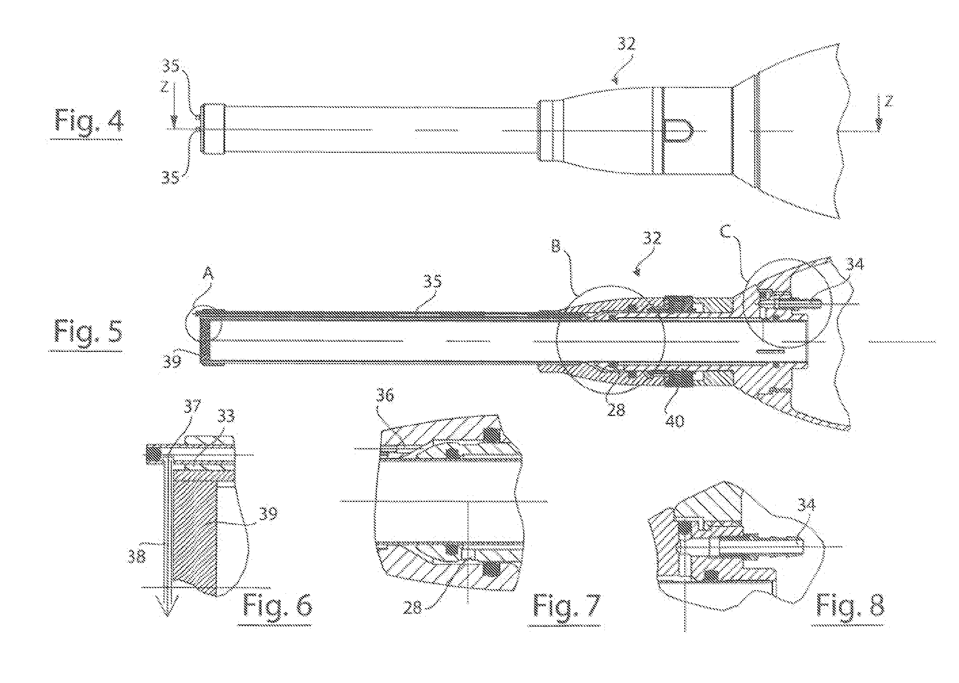

With reference to figures 4-8, the movable sleeve 32 covers the optical tube 12 destined to obtain the vision of the object to be examined and to light the field of view, in the use of the device for work in the oral cavity and using video control. The sleeve 32 is coaxial with the optical tube 12 and the light source is composed of the end 33 of the optical fibres 31, situated around the distal end of the optical tube 12 and sleeve 32.

The sleeve 32 is capable of sliding on the optical tube 12 and being locked with a lock to the distal part with respect to the grip 11 of the video-endoscope 10, and is free to rotate on itself indefinitely. Its inner diameter is greater than the outer diameter of the optical tube 12, so to permit rotation without contact between the outer surface of the optical tube 12 and the inner surface of the sleeve 32. In the air space that is defined between the outer surface of the optical tube 12 and the inner surface of the sleeve 32, pressurised air is made to enter through the orifice 34 and from this subsequently through the holes 28. Such pressurised air prevents contamination.

In addition, on the sleeve 32, ducts 35 are made in which the air enters through the openings 36 and exits at the distal end of the video-endoscope through the opening 37, which conveys an air current 38 onto a protection glass 39 placed at the distal end of the optical tube 12.

Such air flow maintains the protection glass 39 clean, facilitating the vision in the presence of sprays deriving from the use of the instruments; it also avoids the misting due to the patient's breath.

In order to facilitate the installation of the sleeve on the grip 11 of the video-endoscope 10, a connector 40 is moreover provided for.

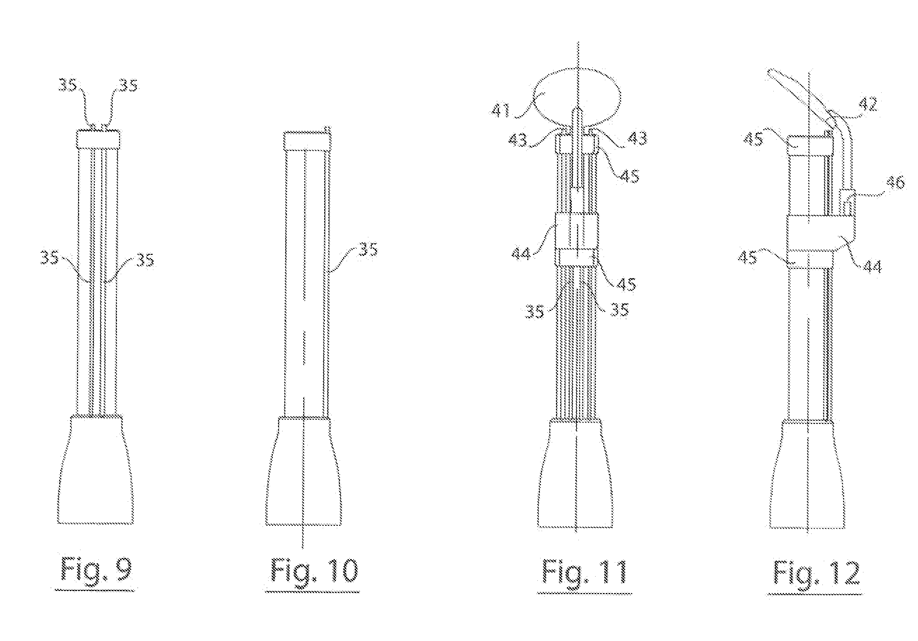

With reference to figures 9 and 10, the tubes 35 outside the optical tube 12 are shown.

With reference to figures 11 and 12, the sleeve 32 moreover bears a mirror 41 at its distal end which forms, with the axis 14 of the optical tube 12, an angle in the range of 30 - 60°. The closest part 42 of the mirror 41 is close to the protection glass 39 and to the ends of suitable tubes 43, which generate an air flow that keeps the surface of the mirror 41 clean.

The mirror 41 is fixed to a movable bush 44 that is locked on the distal part of the sleeve 32, between two end stops 45. The coupling between the mirror 41 and the movable bush 44 is actuated by means of a removable coupling system 46, thus permitting its quick substitution should the surface of the small mirror be scratched.

The sleeve tube 32 (in the version indicated in figure 11) is useful for easily carrying out a rotation, on the control video screen, of the image of the field of view, maintaining the centre of such rotation at the centre of the screen.

The mirror 41 also serves to move aside the patient's cheek and as a small hand-held mirror by the dentist: the light rays deriving from the object to be seen directly by the operator without the video screen. The small mirror 41 can be flat, or concave or convex. Moreover, in order to obtain a more effective cleaning during use inside the oral cavity of the patient, the surface 42 of the glass itself as well as the entire transparent surface of the sleeves of figures 11 and 12 can be coated.

The coatings are obtained by means of suitable technologies, such as PVD (Physical Vapour Deposition), CVD (Chemical Vapour Deposition) or nanostructures in order to obtain a water-repellent effect (also called lotus effect) which reduces the friction between the transparent surface and the water of the sprays.

In order to ensure the stability of the image, the video-endoscope is placed at the end of a compensated, articulated arm.

According to several variant embodiments thereof, the device of the present invention can provide that a plurality of LED light sources are placed directly on the end of the optical tube 12, i.e. that a remote light source is used, from which the light is conveyed up to the end of the optical tube 12 through optical fibres that pass inside the compensated, articulated arm.

In addition, in place of the rotating optical group, the inversion of the image can be obtained by means of related rotation between the video-endoscope and arm, or by the rotation of the CCD catcher, as described in the European patent No. 1355570. Such rotation can be manual or interlocked, or by means of software processing.

In addition, the air tubes for cleaning the protection glass 39 and mirror 41 can be substituted with tubes outside the body of the video-endoscope.

As mentioned above, according to a preferred embodiment, the video-endoscope is fixed on a support to the distal end of a compensated, articulated arm, which permits maintaining it stable position when the arm is left by the operator.

The video endoscope can be easily disconnected from the arm by means of electrical and pneumatic connectors in order to facilitate its maintenance and repair.

The arm is composed of three rigid tubes, as described in the European patent No. 1355570.

The video endoscope can also rotate around its own axis.

Thanks to such degrees of freedom, the video endoscope can be oriented in any position of the dentist work space.

The system is provided with a control card that, in order to facilitate assembly, is preferably fixed on the articulated arm. The control card has the following functions: provide suitable power to the CCD, set the functioning parameters of the CCD, manage the signals in order to obtain the mirror of the image, manage the signals coming from the user interface, acquire and process images. In particular, in a preferred embodiment, the control card permits carrying out the freeze frame.

Finally, the video-endoscope is provided with a user interface, preferably made by means of buttons localised on the rigid tube of the articulated arm. Such buttons have the following functions: system turning on/turning off, image rotation, mirror image, freeze frame, CCD parameter setting, air flow adjustment.

Alternatively, the user interface can be made by means of pedals or by means of commands integrated on the dental drill unit or directly on the body of the video endoscope.

The present invention was described as illustrative but not limiting, according to its preferred embodiments, but it should be understood that variations and/or modifications can be made by those skilled in the art without departing from the related protective scope, as defined by the attached claims.

## Claims

1. Device for medical use and particularly for dental use, of which the main component is a rigid video-endoscope (10) supported by an articulated and compensated arm comprising:
- a rigid video-endoscope (10) with CCD catcher (13), composed of a grip (11) which is extended into an optical tube (12) coaxial therewith, said optical tube (12) being made so to provide an image of the object examined on the plane of the CCD catcher (13), the imaging being directed in the extension of the axis (14) of the video-endoscope (10);
- a sleeve (32) which covers the optical tube (12) and which protects it from the risks of contamination during use, the sleeve (32) being coaxial with optical tube (12), freely rotatable on itself and lockable in the distal part of the grip (11) of the video-endoscope (10), between the sleeve (32) and the optical tube (12) an airspace being defined in which pressurised air is inserted;
- a compensated, articulated arm that bears, fixed to its distal end, a support on which the video-endoscope (10) is locked;
**characterised in that**
- the rotation and reflection of the image is obtained by means of an optical group composed of a first optical half-prism (16), a second optical half-prism (17) and a reflecting body (18) arranged on a rotating housing (19), actuated by a micromotor (22).

2. Device according to claim 1, **characterised in that** the lighting of the field of view is provided by an optical fibre bundle (31), whose opposite end is arranged near a light source (29).

3. Device according to claim 1, **characterised in that** it comprises a system for blowing air on the protection glass (39) placed at the distal end of the optical tube (12).

4. Device according to claim 3, **characterised in that** said blowing system comprises at least one duct (35) with an opening (37) that generates an air flow (38) tangential to the external surface of said protection glass (39).

5. Device according to claim 1, **characterised in that** the sleeve (32) bears, at its distal end, a mirror (41) whose tangent surface forms an angle in the range of 30-60° with the axis (14) of the optical tube (12).

6. Device according to claim 5, **characterised in that** said mirror (41) is fixed to a movable bush (44) that is locked on the distal part of the sleeve (32) between two end stops (45), the coupling between the mirror (41) and the movable bush (44) being actuated by means of a removable coupling system (46).

7. Device according to claims 3, 5 and 6, **characterised in that** the air blow conveyed by said at least one duct (35) is destined to clean the surface (44) of the mirror (41).

8. Device according to claims 3 and 7, **characterised in that** all the surfaces affected by the cleaning air flow, and in particular the surface (42) of the mirror (41), are coated with a suitable water-repellent treatment.
